# EUROPEAN PATENT SPECIFICATION

(11) **EP 2 803 353 B1**
(45) Date of publication and mention of the grant of the patent: **23.05.2018**
(21) Application number: 13004915.8
(22) Date of filing: 14.10.2013
(51) Int. Cl.: A61K 9/28, A61K 31/506

(54) **Compositions of Imatinib**
Zusammensetzungen von Imatinib
Compositions d'imatinib

(30) Priority: 14.05.2013 IN CH21372013
(43) Date of publication of application: 19.11.2014
(73) Proprietor: Hetero Research Foundation, Balanagar Hyderabad Telangana 500 018 (IN)
(72) Inventor: Parthasaradhi Reddy, Bandi, Balanagar Hyderabad Telangana 500018 (IN); Khadgapathi, Podili, Jeedimetla Hyderabad Telangana 500055 (IN); Kamalakar Reddy, Goli, Jeedimetla Hyderabad Telangana 500055 (IN); Kiran Kumar, Madallapalli, Jeedimetla Hyderabad Telangana 500055 (IN)
(74) Representative: Kernebeck, Thomas

(56) References cited:
- WO-A1-2009/042809
- WO-A1-2011/160798
- WO-A1-2013/124774
- WO-A2-2013/008253

## Description

### FIELD OF THE INVENTION

The present invention relates to pharmaceutically acceptable compositions comprising imatinib, more specifically film-coated tablets comprising imatinib mesylate.

### BACKGROUND ART

Imatinib mesylate, 4-[(4-Methyl1-piperazinyl)mefhyl]-N-[4-methyl-3-[[4-(3-pyridinyl)-2-pyrimidinyl]amino]-phenyl]benzamide methanesulfonate, disclosed in EP0564409. Its structural formula as follows:

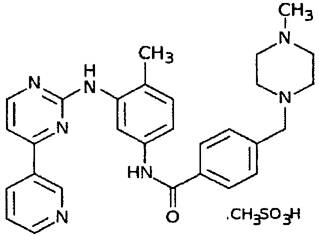

Imatinib mesylate is marketed under the trade name Glivec® in Europe by Novartis in the form of 100 mg and 400 mg tablets for the treatment of various cancers including dermatofibrosarcoma, gastrointestinal stromal tumors and chronic myelogenous leukemia.

Several crystalline polymorphic forms of imatinib mesylate are known. For example, international patent application WO9903854 discloses alpha and beta crystalline forms of Imatinib mesylate. According to WO9903854, beta crystalline form of Imatinib mesylate is more suitable for pharmaceutical applications than alpha form due to the improved stability and flow characteristics of beta form.

For sake of treatment compliance and patient convenience, high drug loaded tablets with a decreased size have been developed.

For instance, , International patent application WO2003090720 discloses film coated tablets prepared by wet granulation comprising from 30% to 80% by weight of imatinib mesylate. Exemplified tablets are coated with aqueous coating dispersion. According to the preferred embodiments disclosed, imatinib mesylate is present in the tablets as beta crystalline form. Further, WO11160798A discloses film-coated tablets with an amount of imatinib mesylate from 82% to 89% which has been wet granulated in water without any further excipient.

Additionally, the anonymous document, "stable tablet formulation containing more than 80% of Imatinib mesylate" IP.COM, reference IPCOM000167554D, 2008, discloses tablets comprising 85% of imatinib mesylate manufactured either by wet granulation, direct compression or roller compaction and coated using a hypromellose based film coat. This document is silent about the crystalline form of imatinib mesylate used in the formulation and the coating solvent.

High drug loaded tablets containing imatinib mesylate in alpha crystalline form have been also disclosed. For example, International patent application WO2012019633 discloses tablets comprising around 60% of alpha imatinib mesylate, compressed from a granulate containing from 95 to 99% of the active ingredient prepared by wet granulation, and coated with an aqueous film-coating solution. Additionally, US2007036850A discloses tablets comprising from 25 to 80% of imatinib mesylate, preferably in the alpha form, made by dry granulation and coated with an aqueous film-forming solution.

International Publication WO2011121593A1 discloses high drug loaded film-coated tablet compositions containing imatinib mesylate in amounts exceeding 80%, wherein the tablets were coated with aqueous-based film coating comprising polyvinyl alcohol.

The present inventors have observed that prior art imatinib high loaded tablet compositions show stability problems when subjected to stress conditions, e.g. polymorphic conversion issues.

Hence, there is a need to develop high drug loaded tablets of imatinib with high stability, particularly which are stable under stress conditions, e.g. stable against polymorphic conversion.

### SUMMARY OF THE INVENTION

The invention provides high drug loaded film coated tablet compositions comprising imatinib mesylate in alpha form, wherein the initial solid form of imatinib mesylate is retained upon storage (e.g. during 1 to 3 months at 40°C±2°C and 75%±5% relative humidity).

The compositions of the invention have unexpectedly shown improved stability over prior art high loaded imatinib tablets, in particular e.g. over those compositions film-coated with aqueous film coating solutions.

Without wishing to be bound by theory, the increased stability may be attributed to the film-coating carried out with organic solvent or mixture of organic solvents comprising hydroxypropyl methylcellulose.

Thus, the inventors have surprisingly found that coating the core of a high drug loaded tablet of alpha imatinib mesylate with a film-forming coating solution in an organic solvent prevents alpha imatinib mesylate from undergoing polymorphic changes upon storage even under stress conditions.

The compositions of the invention have shown other improvements over prior art compositions, e.g. improved dissolution rate.

Therefore, the first aspect of the present invention is directed to an immediate release film coated tablet comprising from 95% to 99% by weight of imatinib mesylate salt based on the total weight of the film-coated tablet, wherein the tablet core comprising the active ingredient and one or more pharmaceutically acceptable excipients, is coated with a solution or dispersion in an organic solvent or mixture of organic solvents of a film coating agent comprising hydroxypropyl methylcellulose and wherein said mesylate salt is in alpha crystalline form characterized by a powder X-ray diffraction (PXRD) pattern having peaks at following 2θ angle positions at about 4.9, 10.5, 14.9, 16.5, 17.7, 18.1, 18.6, 19.1, 21.3, 21.6, 23.2, 24.9, 28.0, and 28.6 ± 0.2 degrees.

The second aspect of the invention is directed to a process for preparing an immediate release film coated tablet comprising from 95% to 99% by weight of imatinib mesylate salt based on total weight of the film coated tablet and one or more pharmaceutically acceptable excipients, comprises coating a tablet core comprising the active ingredient and one or more pharmaceutically acceptable excipients with a solution or dispersion in an organic solvent or mixture of organic solvents of a film coating agent comprising hydroxypropyl methylcellulose and wherein said mesylate salt is in alpha crystalline form characterized by a powder X-ray diffraction (PXRD) pattern having peaks at following 2θ angle positions at about 4.9, 10.5, 14.9, 16.5, 17.7, 18.1, 18.6, 19.1, 21.3, 21.6, 23.2, 24.9, 28.0, and 28.6 ± 0.2 degrees.

### DETAILED DESCRIPTION OF THE INVENTION

The crystalline form alpha of imatinib mesylate is a polymorphic form as prepared and characterized by methods disclosed in WO9903854, more specifically as shown in Example 1 of WO9903854.

Alpha crystalline form of imatinib mesylate is characterized by a powder x-ray diffraction pattern showing its relevant peaks at 2θ angle values of about 4.9, 10.5, 14.9, 16 .5, 17.7, 18.1, 18.6, 19.1, 21.3, 21.6, 23.2, 24.9, 28.0, and 28.6 ± 0.2 degrees.

An immediate-release dosage form, as defined in European Pharmacopoeia 7.0 04/2010:1502, is a preparation showing a release of the active substance which is not deliberately modified by a special formulation design and/or manufacturing method. In the case of a solid dosage form, the dissolution profile of the active substance depends essentially on its intrinsic properties.

By an organic solvent based film coating is meant herein a film coating that is applied employing a coating solvent system consisting of an organic solvent or mixture of organic solvents.

Organic solvent or mixture of organic solvents as described herein may contain up to a 10% (w/w) of water, preferably the solvents are anhydrous. By anhydrous organic solvent it is mean herein an organic solvent that contain less than 5% (w/w) of water.

The immediate release film coated tablet of the invention comprises from 95% to 99% by weight, more preferably from 96% to 99% by weight, of imatinib mesylate.

The immediate release film coated tablet of the invention comprises imatinib mesylate in alpha crystalline form. Advantageously, in said immediate release film coated tablet, said alpha crystalline form of imatinib mesylate is retained upon storage at 40°C and at 75% relative humidity for 3 months, when measured by XRPD technique.

Most preferably, in said immediate release
film coated tablet, imatinib salt does not undergo polymorphic conversion upon storage under stress conditions. The immediate release film coating agent used in the compositions of the invention comprises hydroxypropyl methylcellulose, for example hydroxypropyl methylcellulose based Opadry® product range and mixtures of hydroxypropyl methylcellulose and polyethylene glycol. More preferably, the film coating agent is an Opadry® composition comprising hydroxypropyl methyl cellulose, polyethylene glycollmacrogol and talc.

According to the present invention, the core tablet may comprise pharmaceutically acceptable excipients selected from diluents, glidants, lubricants and combinations thereof. The expression "pharmaceutically acceptable excipients" or simply "excipients" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

Exemplary diluents include microcrystalline cellulose, lactose, starch, pregelatinized starch, calcium carbonate, calcium sulfate, sugar, dextrates, dextrin, dextrose, dibasic calcium phosphate, tribasic calcium phosphate, kaolin, magnesium carbonate, magnesium oxide, maltodextrin, mannitol, polymethacrylates, potassium chloride, powdered cellulose, sodium chloride, sorbitol, talc and the like, and combinations thereof.

Exemplary binders include polyvinyl pyrrolidone, copovidone, hydroxypropyl cellulose, hydroxypropyl methylcellulose, pregelatinized starch, powdered acacia, gelatin, guar gum, carbomers and the like, and combinations thereof.

Exemplary glidants include colloidal silicon dioxide, magnesium trisilicate, powdered cellulose, starch, talc, tribasic calcium phosphate and the like, and combinations thereof.

Exemplary lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, zinc stearate and the like, and combinations thereof. Preferred lubricant is magnesium stearate.

Advantageously, the process of the invention provide small tablets with a high load of drug, maintaining the amount of excipients as low as possible while meeting the requirements of bioavailability and bioequivalence of pharmaceutical products. Therefore, in a preferred embodiment of the invention, the tablet prepared by the process of the invention is free from binders and disintegrants. In a more preferred embodiment, the tablet core of the compositions of the invention, obtained by the process of the invention, consists of imatinib mesylate and lubricants, more preferably magnesium stearate.

In one embodiment the tablet core consists of from 0.10% to 1.00% of magnesium stearate and from 97.75% to 99% of imatinib mesylate, and from 0.75% to 1.25% of HPMC based film-coating by weight percentage of the final film-coated tablet.

For the purposes of the present invention, unless otherwise stated, the term "percentage (%) by weight" refers to the percentage of each ingredient of the composition in relation to the total weight of the final film-coated tablet.

For the purposes of the invention, any ranges given include both the lower and the upper end-points of the range. Ranges given should be considered approximate, unless specifically stated.

According to the process of the invention, the coating process of the tablet core is carried out in an organic solvent or a mixture of organic solvents.

In a preferred embodiment, the organic solvent or mixture of organic solvents used in the process of the invention is selected from C1-C3 alcohol, e.g. methanol, ethanol or isopropyl alcohol, ethyl acetate, ethyl lactate, acetone, methylenechloride, 1,1,1-trichloroethane, chloroform, and mixtures thereof. Preferably, the solvent system is a mixture of C1-C3 alcohol/methylene chloride, more preferably Isopropyl alcohol/methylene chloride.

In a preferred embodiment of the invention, the solvent system of the coating step is anhydrous, i.e. it has a content of water up to 5% in weight of the solvent system.

The coating step of the process of the invention encompasses applying the coating solution to the core tablet and drying the coated tablet for removing the solvent and obtaining the final film-coated tablet by processes and methods known in the art. The appropriate conditions and/or equipment used, can readily be determined by those skilled in the art according to the tablet being prepared.

Similarly, tablet core compositions of the present invention can also be prepared according to methods well known in the state of the art. Most preferred method for preparing the tablet core of the compositions of the invention is by direct compression. The film coated tablet of the invention comprises from 95% to 99% by weight, more preferably from 96% to 99% by weight, of imatinib mesylate.

In a particularly preferred embodiment of the process of the invention, the tablet core is prepared by direct compression of imatinib mesylate, optionally together with one or more pharmaceutically acceptable excipients or carriers. Imatinib mesylate is in alpha crystalline form.

In a still more preferred embodiment, the tablet core is prepared by direct compression of alpha imatinib mesylate and a lubricant.

Direct compression involves blending a composition comprising imatinib mesylate as crystalline alpha form and one or more excipients and compressing it directly into a tablet. Direct compression is easy, simple and applicable for industrial scale. The appropriate conditions and/or equipment used for suitably compressing the blend for obtaining the core tablet can readily be determined by those skilled in the art according to the tablet to be prepared.

With regard to the specific conditions for carrying out the processes of the invention, the skilled person would know how to adjust the parameters of each of the steps indicated above in the light of the description and examples of the present invention.

An immediate-release film-coated tablet obtained by the process of the invention is also disclosed herein.

The immediate-release film-coated tablet "obtained by" the process of the invention is used here to define the composition by the process for obtaining it and refers to the product obtained by the preparation process comprising the coating step as defined above. For the purposes of the invention the expressions "obtainable", "obtained" and equivalent expressions are used interchangeably, and in any case, the expression "obtained" encompasses the expression "obtainable".

All the embodiments of the immediate-release film-coated tablet of the invention contemplate all the combinations provided by all the embodiments of the process of the invention and the combinations thereof.

Further, the invention also encompasses an immediate-release film-coated tablet comprising from 95% to 99%, more preferably from 96% to 99% by weight of imatinib mesylate salt, based on the total weight of the film-coated tablet, and one or more pharmaceutically acceptable excipients, wherein said mesylate salt is in alpha crystalline form and the tablet is prepared by a process comprises coating a core tablet with a film coating agent employing an organic solvent or mixture of organic solvents as coating solvent system.

The immediate-release film-coated tablet obtained by the process of the invention has surprisingly shown improved storage stability maintaining imatinib mesylate stable in crystalline alpha form without undergoing polymorphic conversion upon storage.

Polymorphic conversion is measured by techniques known in the art. Preferably, the percentage of polymorphic conversion is measured by known PXRD techniques. For instance, in a mixture of polymorphs the amount of each polymorph can be calculated with reference to the relative intensity of the unique characterizing XRD peaks of each polymorph.

In a more preferred embodiment of the invention, the immediate-release film-coated tablet obtained by the process of the invention retains alpha crystalline form of imatinib mesylate upon storage at 40°C, 75% RH, for 3 months.

A further aspect of the invention is directed to a composition comprising a tablet core obtained by direct compression of a composition that comprises from 95% to 99%, more preferably from 96% to 99%, of imatinib mesylate based on total weight of the film coated tablet composition and one or more excipients, wherein the tablet core is coated with an organic solvent film coating solution comprising hydroxypropyl methylcellulose.

A further aspect of the invention is directed to a stable film-coated immediate-release tablet composition comprising alpha crystalline form of imatinib mesylate and a pharmaceutically acceptable excipient, wherein the tablet composition retains its initial polymorphic form upon storage (e.g. during 1 to 3 months at 40°C±2°C and 75%±5% relative humidity). More specifically, wherein said film coating is applied as an organic solvent based film coating comprising hydroxypropyl methylcellulose.

A further aspect of the invention is directed to a stable film coated tablet composition comprising, based on total weight of the film coated tablet, from 97% to 99% of alpha crystalline form of imatinib mesylate, 0.1 to 1% of magnesium stearate and from 0.75% to 1.25% of hydroxypropyl methylcellulose based film coating; wherein the composition is free of binders and disintegrants; and the composition is prepared by direct compression process. Preferably, wherein the film coating is an organic solvent based film coating.

Throughout the description and claims the word "comprise" and variations of the word, are not intended to exclude other technical features, additives, components, or steps. Furthermore, the word "comprise" encompasses the case of "consisting of'. Additional objects, advantages and features of the invention will become apparent to those skilled in the art upon examination of the description or may be learned by practice of the invention. The following examples are provided by way of illustration, and they are not intended to be limiting of the present invention.

### EXAMPLES

Following examples are given for the purpose of illustrating the invention and shall not be construed as limiting the scope of the invention.

### Example 1 to 3:

**Table 1:**

| Ingredients | Example 1 mg/tablet | Example 2 mg/tablet | Example 3 mg/tablet |
|---|---|---|---|
| Imatinib mesylate (α-form) | 478.00^{#} | 478.00^{#} | 478.00^{#} |
| Magnesium stearate | 0.75 | 2.00 | 3.00 |

| Coating: | | | |
|---|---|---|---|
| Opadry® yellow 03F82597* | 4.78 | 4.80 | 4.81 |
| Iso propyl alcohol | q.s. | q.s. | q.s. |
| Methylene chloride | q.s. | q.s. | q.s. |
| Coated tablet weight | 483.53 | 484.80 | 485.81 |

| | | | |
|---|---|---|---|
| ^{#} Each 478mg of Imatinib mesylate contains 400mg of Imatinib. ^{*} Opadry® yellow 03F82597 composition comprises hydroxypropyl methylcellulose, iron oxide yellow, polyethylene glycol/macrogol, talc, titanium dioxide and iron oxide red. | | | |

### Brief manufacturing process:

1. Imatinib mesylate was sifted and loaded into blender,
2. magnesium stearate was sifted and loaded into blender and blended for 5 minutes,
3. lubricated blend of step 2 was compressed into tablet,
4. tablet of step 3, was film coated using Opadry® yellow 03F82597 dispersion in isopropyl alcohol and methylene chloride.

### Example 5: (Comparative example):

Comparative dissolution study was conducted between the composition of imatinib prepared according the present invention and composition of imatinib prepared according to the International Publication WO2011121593A1:

### Dissolution conditions:

| | |
|---|---|
| Dissolution Medium | : 0.1N HCl |
| Volume | : 1000 ml |
| Apparatus | : USP II (Paddle) |
| Speed | : 50 RPM |

**Table 2:**

| Time in minutes | Composition of imatinib prepared according to the present invention (Example 3) | Composition of imatinib prepared according to the '593 patent |
|---|---|---|
| 10 | 58 | 54 |
| 15 | 82 | 73 |
| 20 | 94 | 85 |
| 30 | 102 | 98 |
| 45 | 103 | 102 |

As can be seen from Table 2, composition of imatinib prepared as per Example 3 of the present invention showed slightly better dissolution properties as compared to composition of imatinib prepared according to the '593 patent.

### Example 6: (Stability study):

Stability of imatinib tablets prepared as per Example 3 were evaluated based on polymorphic stability after storage for three months at 40°C and 75% relative humidity.
The polymorphic stability refers to the stability of alpha crystalline form of imatinib mesylate to remain in the initial polymorphic form without undergoing polymorphic conversion after storage.

The polymorphic conversion is measured by techniques known in the art. In particular, each known polymorphic form of imatinib may be characterized by a unique set of peaks in the powder x-ray diffraction pattern having 2θ angle positions. Table 3 below shows powder x-ray diffraction pattern of Imatinib tablets (Example 3) stored at 40°C and 75%RH for 3 months

**Table 3:**

| Peaks in the powder x-ray diffraction pattern (XRD) | |
|---|---|
| Initial form of imatinib mesylate, having 2θ angle positions at about (± 0.2 degrees) | After storage for three months, having 2θ angle positions at about (± 0.2 degrees) |
| 4.914 | 4.951 |
| 10.459 | 10.501 |
| 14.913 | 14.949 |
| 16.511 | 16.554 |
| 17.708 | 17.765 |
| 18.107 | 18.164 |
| 18.644 | 18.695 |
| 19.099 | 19.147 |
| 21.298 | 21.348 |
| 21.640 | 21.690 |
| 23.184 | 23.222 |
| 24.911 | 24.941 |
| 28.050 | 28.071 |
| 28.568 | 28.592 |

Results from Table 3, showed that the alpha crystalline form of imatinib mesylate is retained in the tablets that were coated with organic solvent based hydroxypropyl methylcellulose.

### Reference Examples 7 and 8:

**Table 4:**

| Ingredients | Example 7 mg/tablet | Example 8 mg/tablet |
|---|---|---|
| Imatinib mesylate^{#} (amorphous) | 478.00 | 478.00 |
| Lactose monohydrate | 40.00 | - |
| Microcrystalline cellulose | 37.00 | - |
| Povidone K-90 | 5.00 | - |
| Colloidal silicon dioxide | 2.00 | - |
| Magnesium stearate | 5.00 | 3.00 |

| Coating: | | |
|---|---|---|
| Opadry® yellow 03F82597 | 8.00 | 4.81 |
| Iso propyl alcohol | q.s. | q.s. |
| Methylene chloride | q.s. | q.s. |
| Coated tablet weight | 575.00 | 485.81 |

| | | |
|---|---|---|
| ^{#} Each 478mg of Imatinib mesylate contains 400mg of Imatinib. | | |

### Brief manufacturing process:

1. All the ingredients except magnesium stearate were sifted and loaded into blender,
2. magnesium stearate was sifted,
3. blend of step 1, was lubricated with magnesium stearate of step 2,
4. lubricated blend of step 3, was compressed into tablet,
5. tablet of step 4, was film coated using Opadry® yellow 03F82597 dispersion in isopropyl alcohol and methylene chloride.

### REFERENCES CITED IN THE APPLICATION

EP0564409
WO9903854
IPCOM000167554D
WO2003090720
WO11160798
WO2011121593
European Pharmacopoeia 7.0 04/2010:1502

## Claims

1. An immediate release film coated tablet comprising from 95% to 99% by weight of imatinib mesylate salt as an active agent based on the total weight of the film-coated tablet, wherein the tablet core comprising the said active ingredient and one or more pharmaceutically acceptable excipients, is coated with a solution or dispersion in an organic solvent or mixture of organic solvents of a film coating agent comprising hydroxypropylmethyl cellulose and wherein said mesylate salt is in alpha crystalline form **characterized by** a powder X-ray diffraction (PXRD) pattern having peaks at following 2θ angle positions at about 4.9, 10.5, 14.9, 16.5, 17.7, 18.1, 18.6, 19.1, 21.3, 21.6, 23.2, 24.9, 28.0, and 28.6 ± 0.2 degrees.

2. The immediate release film coated tablet according to claim 1, comprising from 96% to 99% by weight of imatinib mesylate salt as an active agent based on the total weight of the film-coated tablet.

3. The immediate release film coated tablet according to claim 1, wherein said alpha crystalline form of imatinib mesylate is retained upon storage at 40°C and at 75% relative humidity for 3 months, when measured by XRPD technique.

4. A process for preparing an immediate release film coated tablet comprising from 95% to 99% by weight of imatinib mesylate salt as an active agent based on total weight of the film coated tablet and one or more pharmaceutically acceptable excipients, comprising the following step:
a) coating a tablet core comprising the said active ingredient and one or more pharmaceutically acceptable excipients with a solution or dispersion in an organic solvent or mixture of organic solvents of a film coating agent comprising hydroxypropylmethyl cellulose
and wherein said mesylate salt is in alpha crystalline form **characterized by** a powder X-ray diffraction (PXRD) pattern having peaks at following 2θ angle positions at about 4.9, 10.5, 14.9, 16.5, 17.7, 18.1, 18.6, 19.1, 21.3, 21.6, 23.2, 24.9, 28.0, and 28.6 ± 0.2 degrees.

5. The process according to claim 4, wherein the immediate release film coated tablet comprises from 96% to 99% by weight of imatinib mesylate salt as an active agent based on the total weight of the film-coated tablet.

6. The process according to claim 4, wherein said alpha crystalline form of imatinib mesylate is retained upon storage at 40°C and at 75% relative humidity for 3 months, when measured by XRPD technique.

7. The process according to any of the claims 4-6, wherein said tablet core consists of imatinib mesylate and lubricant.

8. The process according to any of the claims 4-7, wherein the composition of the film-coated tablet is from 0.10% to 1.00% by weight of magnesium stearate, from 0.75% to 1.25% by weight of HPMC based film coating and from 97.75 to 99% by weight of imatinib mesylate.

9. The process according to any of the claims 4-8, wherein said organic solvent or mixture of organic solvents is selected from the group consisting of methanol, ethanol, isopropanol, ethyl acetate, ethyl lactate, acetone, methylenechloride, 1,1,1-trichloroethane, chloroform, and mixtures thereof.

10. The process according to claim 9, wherein the organic solvent is a mixture of C1-C3 alcohol/methylene chloride, preferably isopropanol/methylene chloride.

11. The process according to any of the claims 4-10, wherein said organic solvent or mixture of organic solvents is anhydrous.

12. The process according to any of the claims 4-11, wherein the tablet core is prepared by direct compression of alpha crystals or amorphous imatinib mesylate, optionally together with one or more pharmaceutically acceptable excipients or carriers.

## Patentansprüche

1. Filmtablette mit sofortiger Freisetzung, die 95 bis 99 Gew.-% Imatinib-Mesylatsalz als Wirkstoff umfasst basierend auf dem Gesamtgewicht der Filmtablette, wobei der Tablettenkern, der den Wirkstoff und einen oder mehrere pharmazeutisch annehmbare Hilfsstoffe umfasst, mit einer Lösung oder Dispersion von einem Filmüberzugsmittel, das Hydroxypropylmethylcellulose umfasst, in einem organischen Lösungsmittel oder in einem Gemisch von organischen Lösungsmitteln beschichtet ist, und wobei das Mesylatsalz in alpha-kristalliner Form vorliegt, **gekennzeichnet durch** ein Pulver-Röntgen-Diffraktogramm (powder X-ray diffraction, PXRD) mit Peaks bei den folgenden 2-theta-Winkelpositionen von etwa 4,9, 10,5, 14,9, 16,5, 17,7, 18,1, 18,6, 19,1, 21,3, 21,6, 23,2, 24,9, 28,0, und 28,6 ±0,2 °.

2. Filmtablette mit sofortiger Freisetzung nach Anspruch 1, die 96 bis 99 Gew.-% Imatinib-Mesylatsalz als Wirkstoff umfasst basierend auf dem Gesamtgewicht der Filmtablette.

3. Filmtablette mit sofortiger Freisetzung nach Anspruch 1, wobei die alpha-kristalline Form des Imatinib-Mesylats nach dreimonatiger Lagerung bei 40 °C und bei 75 % relativer Luftfeuchtigkeit erhalten bleibt gemessen mittels XRPD-Methode.

4. Verfahren zur Herstellung einer Filmtablette mit sofortiger Freisetzung, die 95 bis 99 Ges.-% Imatinib-Mesylatsalz als Wirkstoff, basierend auf dem Gesamtgewicht der Filmtablette, und einen oder mehrere pharmazeutisch annehmbare Hilfsstoffe umfasst, umfassend die folgenden Schritte:
a) Beschichten eines Tablettenkerns, der den Wirkstoff und einen oder mehrere pharmazeutisch annehmbare Hilfsstoffe umfasst, mit einer Lösung oder Dispersion von einem Filmüberzugsmittel, das Hydroxypropylmethylcellulose umfasst, in einem organischen Lösungsmittel oder in einem Gemisch von organischen Lösungsmitteln, wobei das Mesylatsalz in alpha-kristalliner Form vorliegt, **gekennzeichnet durch** ein Pulver-Röntgen-Diffraktogramm (powder X-ray diffraction, PXRD) mit Peaks bei den folgenden 2- theta-Winkelpositionen von etwa 4,9, 10,5, 14,9, 16,5, 17,7, 18,1, 8,6, 19,1, 21,3, 21,6, 23,2, 24,9, 28,0, und 28,6 ±0,2 °.

5. Verfahren nach Anspruch 4, wobei die Filmtablette mit sofortiger Freisetzung 96 bis 99 Gew.-% Imatinib-Mesylatsalz als Wirkstoff umfasst basierend auf dem Gesamtgewicht der Filmtablette.

6. Verfahren nach Anspruch 4, wobei die alpha-kristalline Form des Imatinib-Mesylats nach dreimonatiger Lagerung bei 40 °C und bei 75 % relativer Luftfeuchtigkeit erhalten bleibt gemessen mittels XRPD-Methode.

7. Verfahren nach einem der Ansprüche 4 bis 6, wobei der Tablettenkern aus Imatinib-Mesylat und Gleitmittel besteht.

8. Verfahren nach einem der Ansprüche 4 bis 7, wobei die Zusammensetzung der Filmtablette ist von 0,10 bis 1,00 Gew.-% Magnesiumstearat, von 0,75 bis 1,25 Gew.-% HPMC-basierte Filmbeschichtung und von 97,75 bis 99 Gew.-% Imatinib-Mesylat.

9. Verfahren nach einem der Ansprüche 4 bis 8, wobei das organische Lösungsmittel oder das Gemisch von organischen Lösungsmitteln ausgewählt ist aus der Gruppe bestehend aus Methanol, Ethanol, Isopropanol, Ethylacetat, Ethyllaktat, Aceton, Methylenchlorid, 1,1,1-Trichlorethan, Chloroform und Gemischen davon.

10. Verfahren nach Anspruch 9, wobei das organische Lösungsmittel ein Gemisch von C1-C3 Alkohol//Methylenchlorid ist, vorzugsweise fsopropanol/Methylenchlorid.

11. Verfahren nach einem der Ansprüche 4 bis 10, wobei das organische Lösungsmittel oder das Gemisch von organischen Lösungsmitteln wasserfrei ist.

12. Verfahren nach einem der Ansprüche 4 bis 11, wobei der Tablettenkern durch Direlctverpressen von alpha-Kristallen oder amorphem Imatinib-Mesylat hergestellt ist, optional zusammen mit einem oder mehreren pharmazeutisch annehmbaren Hilfsstoffen oder Trägern.

## Revendications

1. Comprimé pelliculé à libération immédiate, comprenant de 95% à 99% en poids de sel de mésylate d'imatinib en tant qu'agent actif, basé sur le poids total du comprimé pelliculé, dans lequel le noyau du comprimé comprenant ledit ingrédient actif et un ou plusieurs excipients pharmaceutiquement acceptables est enrobé d'une solution ou dispersion, dans un solvant organique ou un mélange de solvants organiques, d'un agent de pelliculage comprenant de l'hydroxypropylméthylcellulose et dans lequel ledit sel de mésylate est sous forme cristalline alpha **caractérisée par** un diagramme de diffraction des rayons X sur poudre (DRXP) ayant des pics suite aux positions des angles 2θ à environ 4,9, 10,5, 14,9, 16,5, 17,7, 18,1, 18,6, 19,1, 21,3, 21,6, 23,2, 24,9, 28,0 et 28,6 ± 0,2 degrés.

2. Comprimé pelliculé à libération immédiate selon la revendication 1, comprenant de 96% à 99% en poids de sel de mésylate d'imatinib en tant qu'agent actif, basé sur le poids total du comprimé pelliculé.

3. Comprimé pelliculé à libération immédiate selon la revendication 1, où ladite forme cristalline alpha du mésylate d'imatinib est conservée après stockage à 40°C et à 75% d'humidité relative pendant 3 moins, lorsqu'elle est mesurée par une technique de DRXP.

4. Procédé de préparation d'un comprimé pelliculé à libération immédiate, comprenant de 95% à 99% en poids de sel de mésylate d'imatinib en tant qu'agent actif, basé sur le poids total du comprimé pelliculé, et un ou plusieurs excipients pharmaceutiquement acceptables, comprenant les étapes suivantes :
a) l'enrobage d'un noyau de comprimé comprenant ledit ingrédient actif et un ou plusieurs excipients pharmaceutiquement acceptables, avec une solution ou dispersion, dans un solvant organique ou un mélange de solvants organiques, d'un agent de pelliculage comprenant de l'hydroxypropylméthylcellulose
ledit sel de mésylate étant sous forme cristalline alpha **caractérisée par** un diagramme de diffraction des rayons X sur poudre (DRXP) ayant des pics suite aux positions des angles 2θ à environ 4,9, 10,5, 14,9, 16,5, 17,7, 18,1, 18,6, 19,1, 21,3, 21,6, 23,2, 24,9, 28,0 et 28,6 ± 0,2 degrés.

5. Procédé selon la revendication 4, dans lequel le comprimé pelliculé à libération immédiate comprend de 96% à 99% en poids de sel de mésylate d'imatinib en tant qu'agent actif, basé sur le poids total du comprimé pelliculé.

6. Procédé selon la revendication 4, dans lequel ladite forme cristalline alpha du mésylate d'imatinib est conservée après stockage à 40°C et à 75% d'humidité relative pendant 3 moins, lorsqu'elle est mesurée par une technique de DRXP.

7. Procédé selon l'une quelconque des revendications 4-6, dans lequel ledit noyau de comprimé est constitué de mésylate d'imatinib et de lubrifiant.

8. Procédé selon l'une quelconque des revendications 4-7, dans lequel la composition du comprimé pelliculé est de 0,10% à 1,00% en poids de stéarate de magnésium, de 0,75% à 1,25% en poids de pelliculage à base de HPMC et de 97,75 à 99% en poids de mésylate d'imatinib.

9. Procédé selon l'une quelconque des revendications 4-8, dans lequel ledit solvant organique ou mélange de solvants organiques est choisi dans le groupe constitué par le méthanol, l'éthanol, l'isopropanol, l'acétate d'éthyle, le lactate d'éthyle, l'acétone, le chlorure de méthylène, le 1,1,1-trichloroéthane, le chloroforme, et leurs mélanges.

10. Procédé selon la revendication 9, dans lequel le solvant organique est un mélange alcool en C1-C3/chlorure de méthylène, de préférence isopropanol/chlorure de méthylène.

11. Procédé selon l'une quelconque des revendications 4-10, dans lequel ledit solvant organique ou mélange de solvants organiques est anhydre.

12. Procédé selon l'une quelconque des revendications 4-11, dans lequel le noyau de comprimé est préparé par compression directe de cristaux alpha ou de mésylate d'imatinib amorphe, éventuellement avec un ou plusieurs excipients ou véhicules pharmaceutiquement acceptables.
